Europäisches Patentamt

(19) European Patent Office

Office européen des brevets

(11) Publication number : **0 465 234 A1**

(12) **EUROPEAN PATENT APPLICATION**

(21) Application number : **91306029.9**

(22) Date of filing : **03.07.91**

(51) Int. Cl.⁵ : **A61K 45/06, A61K 31/00**

(30) Priority : **03.07.90 US 547662**

(43) Date of publication of application :
**08.01.92 Bulletin 92/02**

(84) Designated Contracting States :
**CH DE ES FR GB IT LI**

(71) Applicant : **McNEIL-PPC, INC.**
**Van Liew Avenue**
**Milltown New Jersey 08850 (US)**

(72) Inventor : **Goldman, William J.**
**855 Lewis Lane**
**Ambler, Pennsylvania 19002 (US)**

(74) Representative : **Mercer, Christopher Paul et al**
**Carpmaels & Ransford 43, Bloomsbury Square**
**London WC1A 2RA (GB)**

(54) **Pharmaceutical compositions and methods for alleviating gastrointestinal distress symptoms induced by nonsteroidal anti-inflammatory drugs.**

(57)   This invention relates to an analgesic pharmaceutical composition for treating pain and the symptoms of nonsteroidal anti-inflammatory drug induced gastrointestinal distress comprising an analgesic effective amount of a nonsteroidal anti-inflammatory drug and a gastrointestinal distress relieving effective amount of one or more of an antidiarrheal, antiflatulent, antispasmodic, anticholinergic composition and optionally an antacid in addition thereto and methods of treating the symptoms of nonsteroidal anti-inflammatory drug induced gastrointestinal distress comprising administering such pharmaceutical compositions.

EP 0 465 234 A1

Field of the Invention

This invention relates to novel pharmaceutical compositions for preventing or treating gastrointestinal symptoms induced by nonsteroidal anti-inflammatory drugs. More particularly, the invention comprises treating the gastrointestinal symptoms induced by nonsteroidal anti-inflammatory drugs with a combination of a nonsteroidal anti-inflammatory drug with an antidiarrheal, antiflatulent, antispasmodic, digestive enzymes, and/or anticholinergic composition.

Background of the Invention

Nonsteroidal anti-inflammatory drugs (hereinafter referred to as "NSAID(s)") are known to be effective analgesics and anti-inflammatory agents. While aspirin is a commonly used NSAID for relief of minor aches and pains, nonaspirin NSAIDs provide clinically superior analgesic, anti-inflammatory and antipyretic activity and are particularly well suited for treatment of acute and chronic pain associated with arthritis and other muscular and skeletal disorders. This invention focuses on the therapeutic use of nonaspirin NSAID(s) and providing novel NSAID combinations.

The occurrence of gastrointestinal discomfort associated with the use of nonsteroidal anti-inflammatory drugs is well known (See Pemberton, R.E. and L.J. Strand, "A Review of Upper-Gastrointestinal Effects of the Newer Nonsteroidal Anti-inflammatory Agents", Digestive Diseases and Sciences, 24:53-64, 1979; Silvoso, G.R., K.J. Ivey, J.H. Butt, "Incidence of Gastric Lesions in Patients with Rheumatic Disease on Chronic Aspirin Therapy", Am. Intern. Med.: 91, 517-520, 1979). Ehsanullah and co-workers (See Ehsanullah, R.S.B., M.C. Page, G. Tildesley and J.R. Wood. "Prevention of gastroduodenal damage induced by nonsteroidal anti-inflammatory drugs: controlled trial of ranitidine", BMJ 297:1017-1021, 1988) have indicated that up to 60% of patients on chronic NSAID therapy report dyspepsia as a side effect of treatment. The main toxicity of the NSAIDs occurs in the gastrointestinal tract (See Bollet, A.J., "Nonsteroidal anti-inflammatory Drugs", Textbook of Rheumatology, 2nd Edition, W.B. Saunders Company, p. 761, 1985). Epigastric distress, nausea, vomiting and occasionally more serious side effects such as upper gastrointestinal bleeding are undesirable effects common to almost all studies relating to these drugs.

It is believed that more than 15 million Americans take NSAIDs (See Roth, S.H., NSAID and Gastropathy: A Rheumatologists's Review. J. Rheum. 15: 912-919, 1988). Forty-four percent of all physical disability which occurs in older patients are related to the aches and pains associated with arthritic disorders (See Wilson, D.E. Use of Misoprostol to prevent NSAID-Associated Gastric Ulcers: Geriatric Med. Today 8:82-88, 1989). Occurrence of gastrointestinal side effects accompanying the administration of NSAIDS is common. Such side effects include diarrhea, nausea, flatulence, stomach cramps and pain, and indigestion (these symptoms are collectively referred to and defined hereinafter as "gastrointestinal distress").

While discontinuation of the particular offending NSAID would eliminate gastrointestinal distress problems for many patients, NSAID therapy represents a primary medication in clinical situations where alternative therapy is unacceptable.

The treatment of the aches and pains associated with arthritic disorders or muscular or skeletal disorders, e.g. bursitis or tendinitis, requires the administration of an NSAID. Reduction or treatment of the symptoms of NSAID induced gastrointestinal distress problems can be somewhat mitigated by the co-administration of an antidiarrheal, antiflatulent antispasmodic and/or anticholinergic composition along with the NSAID or the administration of NSAID with an antacid or with food or immediately after meals.

The suggested separate co-administration of NSAIDs with food or other treatment modalities provides obvious practical problems. Adjusting dosage regimens to meal schedules is impractical in a daily routine for patients and may present interaction problems with food and the NSAID which may effect the bioavailability, efficacy or analgesic onset speed of the NSAID. Further, dual administration of separate medications simultaneously presents problems of patient compliance and professional administration particularly, in hospitals and nursing homes.

The concept of combining a nonaspirin NSAID with an agent to lower the gastric acidity of NSAID, reduce acid production associated with its administration and provide a higher alkaline level in order to prevent mucosal damage due to NSAID therapy in a single composition which overcomes the problems of dual administration with either meals or antacids is disclosed in a co-pending patent application of the same inventor filed concurrently herewith. While such a combination, which can be tailored to provide optional amounts of antacids to efficiently and economically buffer NSAIDs, is a significant advance and meets a long felt need for providing a single composition to effectively treat arthritic disorders with an NSAID while mitigating or eliminating NSAID-induced acidity such antacids alone will not effectively reduce other NSAID induced gastrointestinal side effects. In light of the above there exists a long felt need for providing a single composition to effectively treat arthritic disorders with an NSAID while mitigating or eliminating NSAID induced gastrointestinal distress side effects.

## Summary of the Invention

The foregoing object of fulfilling a long felt need for pharmaceutical compositions which can relieve symptoms of pain, e.g. arthritis, by means of NSAIDs while alleviating NSAID induced gastrointestinal distress side effects has now been accomplished in accordance with the compositions and methods of the present invention.

In accordance with the purposes of the invention, as embodied and fully described herein, the invention comprises analgesic pharmaceutical compositions for treating humans suffering from pain and alleviating the gastrointestinal distress symptoms induced by the administration of NSAIDs comprising a combination of an analgesic anti-inflammatory effective amount of a nonaspirin NSAID and a gastrointestinal distress relieving effective amount of a composition selected from the group consisting of antidiarrheals, antiflatulents, antispasmodics, digestive enzymes, anticholinergic compositions and combinations thereof.

In preferred embodiments the NSAID is selected from the group consisting of propionic acid derivatives including ibuprofen, naproxen, fenoprofen, flurbiprofen and ketoprofen; fenamic acid derivatives, including meclofenamate and mefenamic acid; oxicams, including piroxicam; indole acetic acids, including indomethacin, sulindac, tolmetin; and pharmaceutically acceptable salts thereof. The preferred antidiarrheals are selected from the group consisting of loperamide, attapulgite, bismuth subsalicylate, diphenoxylate HCl, polycarbophil, calcium polycarbophil and mixtures thereof. A preferred antiflatulent is simethicone. Preferred antispasmodics are selected from the group consisting of phenobarbital, dicyclomine HCl, belladonna alkaloids,and atropine and salts thereof. Preferred anticholinergic compositions include atropine.

In other preferred embodiments an antacid component is combined with an NSAID and gastrointestinal distress relieving composition preferred antacids including aluminum hydroxide, basic aluminum carbonate, dihydroxyaluminum sodium carbonate, dihydroxyaluminum aminoacetate, calcium carbonate, magnesium carbonate, magnesium hydroxide, magnesium oxide, magaldrate, magnesium trisilicate, sodium bicarbonate, dibasic calcium phosphate, tribasic calcium phosphate, magnesium phosphate and pharmaceutically acceptable salts thereof as well as combinations of the various NSAIDs and various antacids.

In more preferred embodiments ibuprofen or naproxen is used in combination with loperamide.

As embodied and broadly described herein, the invention further comprises a method for treating the symptoms of arthritic disorders and the symptoms of NSAID induced gastrointestinal distress comprising administering a combination pharmaceutical composition to a patient comprising an analgesic, anti-inflammatory effective amount of an NSAID and a gastrointestinal distress relieving effective amount of one or more medicaments described above.

## Detailed Description of Preferred Embodiments of the Invention

Reference will now be made in detail to preferred embodiments of the invention, examples of which are illustrated in the following examples section.

To achieve the object of the invention of providing a pharmaceutical composition for treating the symptoms of pain and especially arthritic disorders and the symptoms of NSAID induced gastrointestinal distress an analgesic, anti-inflammatory effective amount of an NSAID is combined with a gastrointestinal distress relieving effective amount of one or more antidiarrheal, antiflatulent, antispasmodic, digestive enzymes, and/or anticholinergic compositions. In further preferred embodiments one or more antacid(s) may be additionally combined with much compositions.

The symptoms of NSAID induced gastrointestinal distress may include but are not limited to one or more of dyspepsia (e.g. indigestion heartburn and/or epigastric pain), diarrhea, constipation, abdominal distress or pain, stomach cramps, flatulence, nausea, peptic ulcer, and gastroenteritis. The combination composition of the present invention alleviates or relieves the above noted symptoms. For purposes of the present invention the term relieving is defined as reducing, eliminating, controlling, alleviating, or preventing the onset of the symptoms of NSAID induced gastrointestinal distress.

The treatment of various arthritic diseases is related to the reversal of the disease process as well as the symptomatic relief of the pain that accompanies these disorders. This requires the use of NSAIDs which are well-known, clinically proven analgesic and anti-inflammatory agents. Most NSAIDs are weak organic acids that inhibit prostaglandin synthesis. (See Schoen, R.T. and Vender, R.J.: "Mechanisms of Nonsteroidal Anti-inflammatory Drug-induced Gastric Damage." Am. J. Med. 86:449-459, 1989.) The inhibition of prostaglandin synthesis leads to a breakdown of the gastric protective barrier provided by prostaglandin and mucosal damage due in large part to the generation of hydrochloric acid. Concurrently, symptoms of gastrointestinal distress are induced. Attempts to decrease the risks associated with anti-inflammatory treatment by using enteric coated formulations, suppositories, or pro-drugs have not solved this problem (see Ehsanullah, R.S.B., M.C. Page, G. Tildesley, J.R. Wood. "Prevention of gastroduodenal damage induced by nonsteroidal anti-inflammatory drugs: controlled trial of ranitidine." BMJ 297:1017-1021, 1988). Anti-ulcer drugs are often prescribed concomitantly. Since no single agent has been found to be capable of treating the symptoms of arthritic diseases without incurring concomitant gastrointestinal distress and/or injury, a combination composition as is described in this invention can fulfill such long felt need.

The composition of the present invention preferably comprises a combination of the following compositions or their pharmaceutically acceptable salts, an NSAID selected from the group consisting of: propionic acid derivatives including ibuprofen (the term ibuprofen is meant to include administration of both the racemic mixture of R-and S-enantiomers and the substantially pure S-enantiomer which is the analgesic active form of ibuprofen) from 200 to 800 mg per dose; naproxen from 200 to 500 mg per dose; fenoprofen from 200 to 600 mg per dose; flurbiprofen from 50 to 300 mg per dose; ketoprofen from 50 to 300 mg per dose; meclofenamate from 50 to 400 mg per dose; mefenamic acid from 250 to 500 mg per dose; piroxicam from 10 to 20 mg per dose; indomethacin from 25 to 200 mg per dose, sulindac from 150 to 400 mg per dose, tolmetin from 200 to 1200 mg per dose and mixtures thereof; in combination with one or more of the following an effective amount of an antidiarrheal compositions at a preferred dosage range as a component of the composition in accordance with the invention of: loperamide from about 0.5 mg. to 8.0 mg.; attapulgite from about 300 mg. to 1600 mg.; bismuth subsalicylate from about 120 mg. to 1200 mg; diphenoxylate HCl from about 0.7 mg. to 10 mg.; polycarbophil from about 150 to 2000 mg.; and calcium Polycarbophil from about 150 to 2000 mg. Compatible mixtures of these antidiarrheal compositions and their pharmaceutically acceptable salts can also be included in a pharmaceutical composition of the invention.

In other Preferred embodiments of the invention the above described NSAIDS are combined with antiflatulent, antispasmodic, anticholinergic compositions alone or in combination with themselves, antidiarrheal and/or antacid compositions. The preferred dosage ranges for the preferred antiflatulent simethicone is in the range of about 20 to 125 mg. per dosage unit, generally not to exceed 500 mg/day. The dosage ranges may vary for age and weight of a Patient as well as the severity of symptoms. preferred antispasmodics and dosages are selected from the group consisting of phenobarbitol 5 to 100 mg, dicyclomine HCl 5 to 40 mg, belladonna alkaloids 0.05 to 0.50 mg, and atropine 0.01 to 0.10 mg and salts thereof. Preferred anticholinergic compositions include atropine which acts as an antispasmodic as well.

In further embodiments, the combination composition above may be further combined with an antacid including aluminum hydroxide from 300 to 600 mg per dose; basic aluminum carbonate from 500 to 1000 mg per dose; dihydroxyaluminum sodium carbonate from 300 to 600 mg per dose; dihydroxyaluminum aminoacetate from 100 to 2000 mg per dose; calcium carbonate from 600 to 2000 mg per dose; magnesium carbonate from 50 to 2000 mg per dose; magnesium hydroxide from 250 to 2000 mg per dose; magnesium oxide from 250 to 2000 mg per dose; magaldrate from 400 to 800 mg per dose; magnesium trisilicate from 500 to 4000 mg per dose; sodium bicarbonate from 300 to 4000 mg per dose and mixtures thereof.

Loperamide is the most preferred antidiarrheal active for use in the pharmaceutical composition of the invention. Loperamide as a component of the present invention includes pharmaceutically acceptable salts of loperamide such as loperamide HCl. Loperamide acts by slowing intestinal motility and by normalizing water and electrolyte movement through the bowel. Further, loperamide inhibits peristaltic activity by a direct effect on circular and longitudinal muscles of the intestinal walls. Loperamide in man thus prolongs the transit time of the intestinal contents and reduces the daily fecal volume and increases the viscosity and bulk density and thus diminishes loss of foods and electrolytes.

Dosage ranges chosen for the loperamide component of the composition of the present invention depend upon the age and weight of the patient. A preferred adult dose given initially for the treatment of gastrointestinal distress is 4 mg. followed by 2 mg. after each unformed stool until diarrhea is controlled. A preferred ratio of simethicone to loperamide is in the range of from about 100 to 1 to about 10 to 1. Loperamide acts in the intestines and is therefore preferably enteric coating so that it will pass through the stomach and be released in the small intestines. While enteric coating is preferred, it is not essential since loperamide will not be absorbed or metabolized in the stomach but will eventually pass through into the small intestines in any event.

The dosage ranges described above are preferred adult doses and may vary depending upon age and weight of the patient and/or the NSAID administered as would be known by those skilled in the pharmaceutical art. For example, the administrations of piroxicam within the recommended dosage range of 10 to 20 mg may require a smaller dose of gastrointestinal distress relieving composition to provide a gastrointestinal distress relieving effect than would tolmetin in its preferred dosage range of 200 to 1200 mg.

Further, if a combination of, for example mixtures of NSAIDs or antidiarrheals and antacids are used, the dosage for each component part of the mixture may be reduced.

To establish the efficacy of the composition of this invention in humans, patients suffering from the symptoms of arthritic diseases for whom NSAIDs are used and in whom the symptoms of NSAID induced gastrointestinal distress occur can be administered the NSAID with and without the gastrointestinal distress relieving compositions. The patients are then asked to subjectively estimate onset of relief, duration of relief and time to maximum relief or absence of gastrointestinal distress symptoms. Appropriate statistical methods are used to show that NSAIDs/antacid combination medication are efficacious in preventing or reducing the symptoms of NSAID induced gastrointestinal distress.

Other ingredients both active and inactive can be added to the combination pharmaceutical compositions of of the invention. For example, flavoring compositions are desirably added to chewable and liquid dosage forms.

Examples

The invention will now be illustrated by examples. The examples are not intended to be limiting of the scope of the present invention but read in conjunction with the detailed and general description above, provide further understanding of the present invention and an outline of a process for preparing the compositions of the invention. Example 1-10 disclose various formulations for preparing tablets or caplets in accordance with the invention. Various conventional techniques for preparing medicament tablets or caplets can be employed as would be known to those skilled in the art as is disclosed for example by Remington's Pharmaceutical Sciences, Mack Publishing Co., Chapter 90, "Oral Solid Dosage Forms", pp. 1603-1632 (1985). The disclosure of this reference is hereby incorporated herein by reference.

The following examples may be provided in filled gelatin capsule form or as tablets or caplets which include pharmaceutically acceptable tableting aids and excipients as well as optional coloring and flavoring agents in addition to the active ingredients listed below for each of Examples 1-10 and are prepared in accordance with the teaching of "Remington's" or as specifically identified above.

Example 1:

```
200 mg of naproxen
  2 mg of loperamide HCl
```

Example 2:

200 mg of naproxen
240 mg of bismuth subsalicylate

Example 3:

```
200 mg of naproxen
  2 mg of loperamide
 50 mg of magnesium carbonate
```

Example 4:

200 mg of naproxen
300 mg of polycarbophil
300 mg of aluminum hydroxide

Example 5:

```
200 mg of ibuprofen
  2 mg of loperamide
```

Example 6:

    200 mg of ibuprofen
    .025 mg of atropine sulfate
    100 mg of calcium carbonate

Example 7:

$$100 \text{ mg of indomethacin}$$
$$2 \text{ mg of loperamide}$$

Example 8:

Bi-layered Naproxen-Loperamide HCl 2 mg./Simethicone 80 mg., Chewable Tablet

| Ingredients | mg/tablet |
|---|---|
| **SIMETHICONE LAYER** | |
| dicalcium phosphate, NF | 784.000 |
| colloidal silicon dioxide, NF | 40.000 |
| simethicone, USP | 80.000 |
| aspartame, NF | 5.000 |
| flavors | 16.056 |
| stearic acid, NF | 18.879 |
| Layer Total | 943.935 |
| **NAPROXEN-LOPERAMIDE LAYER** | |
| naproxen, USP | 200.000 |
| loperamide HCl, USP | 2.000 |
| sucrose, NF | 112.000 |
| mannitol, USP | 565.120 |
| aspartame, NF | 2.820 |
| flavors | 9.060 |
| stearic acid, NF | 6.000 |
| colloidal silicon dioxide, NF | 3.000 |
| Layer Total | 900.000 |
| Bi-layer Tablet Total | 1843.935 |

Manufacturing Instructions

A. Simethicone Granulation

Combine dicalcium Phosphate, colloidal silicon dioxide, simethicone, aspartame, flavors and stearic acid. Mix using an appropriate mixer (e.g., pK Blender) for 10 minutes.

B. Naproxen-Loperamide Granulation

Granulate naproxen and loperamide HCl with sucrose and a portion of the mannitol using an appropriate fluid bed granulator (i.e., Glatt GPCG-3).

Dry blend stearic acid, colloidal silicon dioxide, aspartame, flavors and the remaining mannitol with the above granulation. Mix for 10 minutes in an appropriate mixer (e.g., pK Blender).

C. Compression

Compress the naproxen loperamide and simethicone granulations as separate layers using a bi-layer tablet press (e.g. Stokes Versa Press).

Example 9

Bi-layer Naproxen-(200 mg)-Loperamide HCl 2 mg./Simethicone 40 mg. Swallowable Caplet

| Ingredients | mg/tab |
|---|---|

## SIMETHICONE LAYER

| | mg/tab |
|---|---|
| dicalcium phosphate, NF | 400.000 |
| colloidal silicon dioxide, NF | 30.000 |
| simethicone, USP | 40.000 |
| sodium starch glycolate, NF | 50.360 |
| stearic acid, NF | 10.090 |
| Layer Total | 530.450 |

## NAPROXEN-LOPERAMIDE LAYER

| | mg/tab |
|---|---|
| naproxen, USP | 200.000 |
| loperamide HCl, USP | 2.000 |
| mannitol, USP | 101.000 |
| sucrose, NF | 112.000 |
| microcrystalline cellulose, NF | 16.460 |
| sodium starch glycolate, NF | 8.880 |
| stearic acid, NF | 1.290 |
| colloidal silicon dioxide, NF | 0.646 |
| Layer Total | 444.276 |
| **Bi-layer Caplet** Total | 974.726 |

Manufacturing Instructions

A. Simethicone Granulation

Combine dicalcium phosphate, colloidal silicon dioxide, simethicone, sodium starch glycolate and stearic acid. Mix using an appropriate mixer (e.g., PK Blender) for 10 minutes.

B. Naproxen-Loperamide Granulation

Granulate naproxen and loperamide HCl with sucrose and a portion of the mannitol using an appropriate fluid bed granulator (e.g., Glatt GPCG-3).

Dry blend stearic acid, colloidal silicon dioxide, microcrystalline cellulose and sodium starch glycolate with the above granulation and mix for 10 minutes using an appropriate mixer (e.g., PK Blender).

C. Compression

Compress the loperamide and simethicone granulations as separate layers using an appropriate bi-layer tablet press (e.g., Stokes Versa Press).

Example 10

Loperamide 2 mg/Ibuprofen 80 mg. Emulsion

### Ingredients

| | |
|---|---:|
| sucrose, NF | 35.00 |
| sorbitol, USP (70%) | 20.00 |
| sodium benzoate, NF | 0.10 |
| benzoic acid, USP | 0.10 |
| citric acid, USP (Anydrous) | 0.032 |
| propylene glycol, USP | 15.00 |
| glycerin, USP | 15.00 |
| carboxy polymethylene, NF | 0.20 |
| loperamide HCl, USP | 0.02 |
| ibuprofen | 0.80 |
| 10% sodium hydroxide solution | 0.80 |
| purified water, USP, qs to: | 100.00 ml |

Manufacturing Instructions

Combine the above ingredients (except 10% sodium hydroxide solution) with mixing.
Add with gentle mixing, 10% sodium hydroxide solution.
QS to final volume with purified water and mix (e.g. IK-Werk mixer at low speed).

Various other dosage forms can be applied herein such as a sustained release, liquid emulsion/suspension or medicament coated chewable tablet form employing the dosage actives provided above or other dosage amounts in accordance with the present invention. For example, the above-noted combination may be formulated in accordance with the teachings of U.S. Patent No. 4,806,359 for sustained release tablets or U.S. patent No. 4,851,226 for chewable tablets. The entire disclosures of these references are hereby incorporated herein by reference.

Method of Treating Patients for the Symptoms of Arthritis Disease With NSAIDs and preventing NSAID Induced Gastrointestinal Distress

A patient exhibiting the symptoms or suffering from the symptoms of arthritic disease treated with an NSAID exhibiting the symptoms of NSAID induced gastrointestinal distress may be treated by the oral administration of one tablet or dosage amount of the pharmaceutical composition in accordance with any of Examples 1-10 to alleviate the specific symptoms associated with each patient. For example, a patient who suffers from diarrhea when treated with an NSAID may be treated with any dosage form of Examples 1-5 and 7-10.

The scope of the present invention is not limited by the description, examples and suggested uses herein and modifications can be made without departing from the spirit of the invention. For example, the pharmaceutical compositions of the invention may be provided in a bi-layer immediate/sustained release formulation for prolonged and/or nighttime treatment of the symptoms of arthritic disease treated with NSAIDs resulting in NSAID induced gastrointestinal distress. Application of the compositions and methods of the present invention for medical and pharmaceutical uses can be accomplished by any clinical, medical and pharmaceutical methods and techniques as are presently or prospectively known to those skilled in the art. Thus it is intended that the presently claimed invention cover the modifications and variations of this invention provided that they come within the scope of the appended claims and their equivalents.

**Claims**

1.  A pharmaceutical analgesic composition, for alleviating gastrointestinal distress symptoms induced by the administration of nonsteroidal anti-inflammatory drugs to humans suffering from pain, comprising a combination of an effective analgesic amount of a nonaspirin nonsteroidal anti-inflammatory drug (NSAID) and an effective gastrointestinal distress relieving amount of one or more of an antidiarrheal, an antiflatulent, an antispasmodic or an anticholinergic.

2.  The pharmaceutical composition of Claim 1, wherein the nonsteroidal anti-inflammatory drug is a propionic acid derivative, a fenamic acid derivative, an oxicam, an indole acetic acid or a pharmaceutically acceptable salt thereof and is preferably ibuprofen, flurbiprofen, fenoprofen, naproxen, ketoprofen, meclofenamate, mefenamic acid, piroxicam, indomethacin, sulindac, tolmetin or a pharmaceutically acceptable salt thereof and is most preferably naproxen, ibuprofen or indomethacin.

3.  The pharmaceutical composition of Claim 2, wherein the NSAID comprises ibuprofen from 200 to 400 mg per dose; naproxen from 200 to 500 mg per dose; fenoprofen from 200 to 500 mg per dose; flurbiprofen from 50 to 300 mg per dose; ketoprofen from 50 to 300 mg per dose; meclofenamate from 50 to 400 mg per dose; mefenamic acid from 250 to 500 mg per dose; piroxicam from 10 to 20 mg per dose; indomethacin from 25 to 200 mg per dose; sulindac from 150 to 400 mg per dose; or tolmetin from 200 to 1200 mg per dose.

4.  The composition of any one of Claims 1 to 3, wherein the non-NSAID composition is selected from: the antidiarrheal compositions loperamide, attapulgite, bismuth subsalicylate, diphenoxylate, polycarbophil or calcium polycarbophil; the antiflatulent composition simethicone; the antispasmodic compositions phenobarbital, dicyclomine, a belladonna alkaloid or atropine; the anticholinergic composition atropine; or a pharmaceutically acceptable salt or mixture thereof.

5.  The pharmaceutical composition of Claim 4, wherein the non-NSAID composition is selected from: the antidiarrheal compositions 0.5 to 0.8 mg of loperamide, 300 mg to 1600 mg of attapulgite, 120 mg to 1200 mg of bismuth subsalicylate, 0.7 mg to 10 mg of diphenoxylate HCl, 150 mg to 2000 mg of polycarbophil, or 150 to 2000 mg of calcium polycarbophil per dose, or a pharmaceutically acceptable salt thereof; the antiflatulent composition 20 to 125 mg of simethicone per dose; the antispasmodic compositions 5 to 100 mg phenobarbital, 5 to 40 mg dicyclomine, 0.05 to 0.50 mg of a belladonna alkaloid, or 0.01 to 0.10 mg atropine, per dose; and the anticholinergic composition 0.01 to 0.10 mg atropine per dose.

6.  The pharmaceutical composition of any one of Claims 1 to 5 comprising:
    a combination of naproxen and loperamide; or
    a combination of naproxen and bismuth subsalicylate.

7.  The pharmaceutical composition of any one of Claims 1 to 6, which is in an oral tablet, caplet, chewable dosage or liquid dosage form.

8.  The pharmaceutical composition of any one of claims 1 to 6, which is in the form of a bi-layer tablet or caplet comprising one layer comprising the NSAIB and loperamide and a second layer comprising simethicone or an antacid.

9.  The pharmaceutical composition of any one of the claims 1 to 8, for use in the analgesic treatment with a nonsteroidal anti-inflammatory drug of a human patient suffering from pain and to alleviate one or more symptoms of nonsteroidal anti-inflammatory drug induced gastrointestinal distress.

**European Patent Office**

# EUROPEAN SEARCH REPORT

Application Number

EP 91 30 6029

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-174006 (RICHARDSON-VICKS) <br> * page 3, lines 6 - 25 * | 1-9 | A61K45/06 <br> A61K31/00 |
| A | BIOSIS PREVIEWS DATABASE <br> BIOSIS, Philadelphia, PA. <br> KOCIAN J. : "Advances in the treatment of diar-rhea." <br> & CESK.GASTROENTEROL. VYZ. 38(6), 1984, p 334-7 <br> Abstract nb: 79079939 <br> * the whole abstract * | 1-9 | |
| A | GB-A-2033915 (AMERICAN HOME PRODUCTS) <br> * abstract * | 1-9 | |
| A,P | EP-A-395431 (MC NEIL PPC) <br> * page 3, lines 47 - 54 * | 6 | |

TECHNICAL FIELDS SEARCHED (Int. Cl.5 )

A61K

The present search report has been drawn up for all claims

| Place of search | Date of completion of the search | Examiner |
|---|---|---|
| BERLIN | 15 OCTOBER 1991 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)